# EUROPEAN PATENT SPECIFICATION

(11) **EP 4 062 903 B1**
(45) Date of publication and mention of the grant of the patent: **18.02.2026**
(21) Application number: 19838717.7
(22) Date of filing: 10.07.2019
(51) Int. Cl.: A61K 35/64, A61K 9/02, A61K 9/20, A61K 9/48, A61P 13/08

(54) **METHOD FOR PRODUCING AN ACTIVE COMPLEX FROM LYMANTRIA DISPAR LARVAE**
VERFAHREN ZUR HERSTELLUNG VON EINEM AKTIVEN KOMPLEX AUS LYMANTRIA-DISPAR-LARVEN
PROCÉDÉ DE FABRICATION DE UN COMPLEXE ACTIF À BASE DE LARVES DE LYMANTRIA DISPAR

(30) Priority: 19.07.2018 MD 20180055
(43) Date of publication of application: 28.09.2022
(73) Proprietor: Ciuhrii, Ceslav, 2021 Chisinau (MD)
(72) Inventor: Ciuhrii, Ceslav, 2021 Chisinau (MD)
(74) Representative: Puscasu, Dan
(86) International application number: PCT/MD2019/000004
(87) International publication number: WO 2020/017944

(56) References cited:
- MD-B1- 4 296
- MD-B1- 4 845
- MD-G2- 2 788
- RO-B1- 122 332
- RO-B1- 122 332
- RO-B1- 123 368
- RO-B1- 130 740
- RU-C1- 2 117 701
- RU-C1- 2 639 479
- SU-A1- 577 001
- ION DUMBRAVEANU ET AL: "Anti-inflammatory activity of Adenoprosin in nonbacterial prostatitis", vol. 60, no. 4, 1 December 2017 (2017-12-01), pages 3 - 9, XP093033082, ISSN: 2537-6373, Retrieved from the Internet <URL:https://zenodo.org/record/1105101/files/MMJ-60-4-1105101.pdf?download=1> DOI: 10.5281/zenodo.1105101
- ANONYMOUS: "Adenosin - instructions for use LP-004871", 18 June 2018 (2018-06-18), pages 1 - 4, XP055759467, Retrieved from the Internet <URL:https://medi.ru/instrukciya/adenoprosin_14831> [retrieved on 20190924]

## Description

The invention relates to the field of pharmaceutics, and namely to the obtaining of an active anti-inflammatory complex from Lymantria dispar larvae, for the treatment of the benign prostatic hyperplasia, whether or not associated with the chronic prostatitis.

It is known the entomological active complex in the Vaseline oil, obtained from the biomass of *Tenebrio Molitor* larvae, containing 45-80% of the total entomological extract and 55-20% of Vaseline oil, effective in the treatment of the prostate diseases, characterized by a high specific antioxidant activity: the ability to inhibit the lipid protein peroxidation (CI50 % = 16 mg/ml) and the ability to inhibit the nitroxide radical (CI50% = 20 mg/ml). For the obtainment of the active complex it is applied a complex technological process that includes: growing of *Tenebrio Molitor* insects in the controlled conditions on an artificial standardized environment; four-time washing of the larvae by four volumes of distilled water; disinfection of larvae by ultraviolet rays; freezing of larvae at -18 °C; defrosting of larvae by means of the gradual increase of the temperature up to the indoor temperature; homogenization of biomass in the homogenizer with stainless steel knives, at the speed of their rotation of 5000 rotations per minute up to the obtainment of the particles of 9-12 microns in size; extraction of water-soluble components from the entomological biomass by the distilled water; separation of the extract by means of vacuum filtration; concentration of the extract by means of evaporation in the vacuum up to the volume of 250 ml; extraction by 70% ethyl alcohol of the alcohol-soluble components; separation of the extract by means of the vacuum filtration, concentration of the extract by means of evaporation in the vacuum at 40 ° C up to the residual volume of 100 ml; combination of two extracts; lyophilization of the entire extract; conditioning of the total extract in the form of the entomological active complex in the Vaseline oil with the following composition: total lyophilized entomological extract - 45 ... 80%; Vaseline oil - 55 ... 20%. The entomological active complex is shaped into a pharmaceutical product - suppositories, which contain 250 mg of the entomological active complex and 1750 mg of lipids [1], (patent MD 4296).

The disadvantage of this active complex and the technology of its obtainment consists in the fact that the technological process includes several critical points that can jeopardize the quality of the active complex. This is the stage of the concentration by means of the evaporation in the vacuum of the aqueous and alcoholic extract. In both cases, a significant heating occurs, which leads to the damage of the components of the entomological extracts that are sensitive to the increase of temperature.

It is known also the anti-inflammatory and antioxidant entomological preparation for the treatment of the benign prostatic hyperplasia and method of its confection [2], (Patent MD 2788).

According to the description, the anti-inflammatory and antioxidant entomological preparation obtained from the larvae of *Lepidoptera* insects, genus *Lymantria* by means of their grinding in the physiological saline up to the formation of a homogeneous mass, filtration and removal of water by means of lyophilization, contains the following components: proteins - 265,0 ... 292,0 mg/g; lipids -140,0 mg/g, cholesterol - 0,1 mg/g; triglycerides - 90,0 mg/g, amylase - 5,1 IU/g, lipase - 100,0 IU/g, antioxidants - 16,86 mg/g, indispensable and semi-substituted amino acids - 379,7 mg/g. Antioxidant effect of the preparation is manifested in the inhibition of the peroxidation of lipids and is determined by the water-soluble antioxidant compounds [2].

The disadvantage of this method is that the procedure of the cultivation of insects for the obtainment of the biomass of larvae is not indicated, the active substance is extracted by water, therefore the preparation contains only the water-soluble antioxidant components.

Also, it is known a method of obtainment of the entomological preparation from the biomass of the first-instar larvae of *Lymantria dispar,* by means of water-alcohol extraction. Larvae are grown in the laboratory conditions on an environment consisting of wheat flour, corn grits, dry plant material, milk powder, dried yeast, glycerine and honey. The collected biomass of the first-instar larvae of *Lymantria dispar* is washed four times by 4 volumes of distilled water. After that, the biomass is dispersed for 15 minutes in a solution of NaCl, 0,9%. The larvae are frozen. The frozen biomass is defrosted, gradually bringing it to the indoor temperature. Using the stainless-steel knife mill, the biomass is mixed at a speed of 5000 rotations per minute and disintegrated up to the obtainment of the particles of 9-12 microns in size. The resulting mass is subjected to the extraction of the biologically active substances by the 20% hydro-ethanol solution. Extraction lasts 60 minutes under the conditions of the constant temperature (18-20 °C) at the constant shaking on the orbital shaker at the speed of 280 rpm (alternatively 240-320 rotations per minute). The extract is separated from the biomass by means of vacuum filtration, followed by the lyophilization of the extract [3], (Ciuhrii V. Biotechnology of the obtainment of pharmaceutical form for the treatment of the benign prostatic hyperplasia. Dissertation for the scientific degree of the candidate of biological sciences. Chisinau, 2019, 129 pages).

The pharmaceutical product is obtained in the form of the rectal suppositories containing 60% of the lyophilized extract.

The disadvantage of the active complex is that it consists only of the components of biomass that can be extracted by the 20% hydro-ethanol solution and, therefore, most of the fat-soluble components are absent in it.

Another disadvantage of the method of the obtainment of the active complex is that as the raw material for the extraction it is used the biomass of the first-instar larvae, which are characterized by small sizes and weight that implies the increase of the prime cost of the final technology; a single extraction by hydro-ethanol is carried out 60 minutes long, as a result some active components of the biomass remain unused. a method for producing an active complex from *Lymantria dispar* larvae.

It is also known a method of the obtainment of the entomological preparation of pupae and larvae of the last instar (the 5^{th} or 6^{th} depending on sex) and eggs of *Lymantria dispar,* i.e. an active complex, that is included in the pharmaceutical composition in the form of the rectal and vaginal suppositories for the treatment of haemorrhoids, prostate adenoma, uterine fibroids [4], (patent RO 122332). According to the indicated source, the manufacture of the pharmaceutical composition includes the following working stages: collection of pupae and larvae of the last instar *Lymantria dispar* from the trunks of the affected oak and poplar trees since June and of eggs - in August-September or growing of larvae and pupae in the laboratory conditions; washing of biological material and its resuspension in the physiological solution; filtration and drying of biological material; if necessary - freezing of material for the purpose of storage until the moment of the further use; grinding of material up to the obtainment of the particles with dimensions of 9-12 microns; filtration through the Bucket filter having 200 apertures per cm2; vacuum spraying of the obtained extract, at the temperature of 130-140 °C at the input and 85-90 °C at the output, or lyophilization in the course of 24-30 hours - the active substance for the production of the pharmaceutical form; preparation of pharmaceutical composition consisting of 1% of active substance in the form of the extract of *Lymantria dispar,* 82% of cocoa butter, 10% of distilled water, 2,68% of helix, 2% of laurate, 1,3% of 2-bromo-nitropropane, 1% of polyglyceryl-4-cetylisostearate PEG/PPG dimethicane 10/1 and 0,02% of dial; conditioning of this formula by melting and homogenization of the obtained mixture at the temperature of 60 °C and pouring into the moulds for the rectal or vaginal suppositories; packing and subsequent storage.

The disadvantage of this method is that as biological raw material are used three stages of the development of insect - eggs, larvae of the last instar and pupae. The biochemical composition of larvae, pupae and eggs differs significantly. The biological activity of these types of biomasses also differs significantly. For example, the antiradical activity of the larval extract is 5-7 times higher than the activity of eggs and pupae [3].

It was also demonstrated that with aging the biological activity of larvae decreases, for example, the antiradical activity of larvae of the last instar is at least 30% lower compared with the activity of larvae of the first instar. Thus, another disadvantage of the closest solution is the use of larvae of the last instar that are characterized by the lowest antioxidant activity [3].

At the same time the source selected as the closest solution does not indicate the proportions for the obtainment of the mixture of larvae and pupae. Proceeding from their different activity, in case of the use of different proportions of larvae and pupae, the products with different biological activity will be obtained. Also, the selected source does not include the description of the process of growing of larvae and pupae in the laboratory conditions that may be the factor influencing the change of the amount and biological properties of the obtained biological material. The aforementioned makes impossible the control of the composition of the active substance.

The document RU2117701 C1 also discloses an active complex made from *Lymantria dispar* larvae, where all the biomass of third and fourth instar larvae is used as the active complex [5] and the document: SU577001 A1 discloses a nutrient medium for cultivating gypsy moth caterpillars *(Lymantria dispar)* in artificial conditions, comprising various components taken in a specific ratio [6].

It must be mentioned that the nutrient medium for the cultivation of the *Lymantria dispar* pupae and larvae is essential for the efficiency of a method for the obtaining of an active complex from *Lymantria dispar* larvae.

The problem solved by the given invention consists in the improving of the efficiency of a method for the obtaining of an active complex from *Lymantria dispar* larvae by establishing an adequate
nutrient medium for the cultivation of the *Lymantria dispar* pupae and larvae and ensuring also
the preservation of the integral biochemical composition of the raw material, for the obtaining of a product with a very high degree of homogeneity and quality.

The proposed method, for the obtaining of an active complex from *Lymantria dispar* larvae of the 3^{rd}, 4^{th} age, includes a sequence of all technological stages: growing of insects, obtainment of active complex and the storage of the active complex under the conditions of refrigerator, for the obtainment of various forms of pharmaceutical preparations including a reasonable amount of active substance having the anti-inflammatory effect, effective for the treatment of the diseases of the prostatic gland. The nutrient medium for the obtaining of *Lymantria dispar* larvae and pupae, according to the invention, consists in: wheat flour, 80-95 g; corn flour, 82-87 g; dry plant material, 110-120 g; skimmed milk powder, 2,5-3,5 g; bee honey, 17-19 g; glycerine, 10-12 g; pinobanksin, 0,01-0,02 g; pinocembrin, 0,01-0,02 g; purified water, 696,5-663,5 ml.

The freezing of larvae and the storage of the obtained of active complex are realised at -18 °C and the grinding of active complex is realised at an extremely low temperature of -55 °C.

The resulting method presents the advantage that it allows the obtaining of an active complex from *Lymantria dispar* larvae by an adequate nutrient medium, ensuring also the preservation of the integral biochemical composition of the raw material, for the obtaining of a product with a very high degree of homogeneity and quality.

The invention is presented in detail below.

The essence of the invention consists in the fact that it is proposed the method of the obtainment of the active complex from *Lymantria dispar* larvae, of the 3rd, 4th age, which includes:
• Cultivation of *Lymantria dispar* in the artificial conditions on a standard medium of the following composition (per 1 kg of medium):

| | |
|---|---|
| Wheat flour | 80-95 g |
| Corn flour | 82-87 g |
| Dry plant material | 110-120 g |
| Skimmed milk powder | 2,5-3,5 g |
| Bee honey | 17-19 g |
| Glycerine | 10-12 g |
| Pinobanksin | 0,01-0,02 g |
| Pinocembrin | 0,01-0,02 g |
| Purified water | 696,5-663,5 ml |

• Freezing of larvae at -18 °C;
• Washing of larvae;
• Primary grinding of larvae - obtainment of active complex;
• Lyophilization of active complex.
• Grinding of active complex at an extremely low temperature (-55 °C);
• Homogenization of active complex (combination of fractions);
• Packaging of active complex;
• Storage of active complex under the conditions of refrigerator (-18 °C).

The technical result of the invention compared with the closest solution is that the method of the obtainment of the active complex allows using the general biochemical composition of the lymantria larvae of the 3^{rd}, 4^{th} age, and the process of the grinding of the biomass of larvae after the lyophilization is carried out at the extremely low temperature (-55 °C) that ensures the preservation of the biochemical composition of the raw material and the obtainment of the product with a very high degree of homogeneity.

The technical result of the invention is achieved due to the use of a full range of biologically active substances of the biomass of lymantria larvae of the 3^{rd}, 4^{th} age and the exclusion of the stages that can lead to the destruction of the thermally sensitive components of the active complex.

According to a specific example of the invention, the method includes the cultivation of the insects of the species *Lymantria dispar,* by the following stages:
1. **Creation of the conditions for the passage of the life cycle of insects in artificial conditions.** Insects are grown in the premises with the area of 15-20 m². The walls are painted with a special resin, which ensures the possibility of the disinfection with the disinfectant solutions. Along the perimeter of the room there are the shelves with the dark Rubbermaid drawers. The temperature is maintained at 27 °C, and humidity is 75%. Photoperiodism is also provided.
2. **Cultivation of insects.** 300-500 adult specimens are placed in the Rubbermaid drawer and fed exclusively with the high-quality wheat bran. With the onset of the hatching, the first-instar larvae are collected and placed in the Rubbermaid drawers with a nutrient medium that has the following composition:

| | |
|---|---|
| Wheat flour | 91,45 g |
| Corn flour | 85,55 g |
| Dry plant material | 117,99 g |
| Skimmed milk powder | 2,95 g |
| Bee honey | 17,70 g |
| Glycerine | 11,80 g |
| Pinobanksin | 0,015 g |
| Pinocembrin | 0,015 g |
| Purified water | 673,33 ml |

The larvae are cultivated in the course of 21 days since the moment of hatching, with the successive transfers to fresh nutrient medium.

3. **Obtainment of active entomological complex from *Lymantria dispar* larvae of the** 3^{rd}, **4^{th} age.** Larvae are collected with the help of tweezers and transferred to special containers for freezing. Larvae are frozen at -18 °C. In such form the raw material can be stored in the course of 24 months and used as necessary.

Before use, the larvae are washed twice by four volumes of 0,9% saline solution and two more times by 4 volumes of the purified water. This procedure allows removing the fibres of the outer shells of the larvae.

Subsequently, the biomass is subjected to grinding in the grinder equipped with the stainless steel knives up to the obtainment of the particles the sizes of which do not exceed 2 mm. The resulting active complex is subjected to the process of lyophilisation.

The last stage consists in the grinding of the active complex in the cryogenic mill at the extremely low temperature that ensures the obtainment of the dispersed particles up to 200 microns in size.

The active complex obtained by the above-described method is packed hermetically in the aluminium pouches and stored until use (up to 24 months) under the conditions of the freezer (t = -18 ± 3 °C).

The results obtained within the framework of the claimed method were compared with the results obtained in accordance with the closest solution. In the latter case (the closest solution) the larvae were grown under the laboratory conditions on the declared medium (p. 2 of the example), the larvae of the last instar (the 5^{th} or 6^{th}) were collected on the 35^{th} day after the larvae hatched from egg.

### Amount of biomass and anti-inflammatory activity of entomological complex according to the claimed method and in accordance with the closest solution

| **Parameter** | **In terms of the closest solution** | **In terms of the claimed method** |
|---|---|---|
| Amount of biomass of larvae, mg, for 1 g of the used medium | 51,07±0,74 | 53,96±1,87* |
| Decrease of the level of the released IL8 (% of decrease in relation to control) | 27,75±3,15 | 42,02±2,63* * |

| | | |
|---|---|---|
| *P=0,047 (<0,05); **P=0,004 (<0,005) | | |

The table demonstrates that the proposed method is more effective in terms of the accumulation of the biomass per gram of the used nutrient medium. Thus, per 1 g of medium in the claimed method were obtained 53,96 ± 1,87 mg of larvae and in the closest solution - 51,07 ± 0,74.

The amount of the released IL8 was reduced by the active complex obtained in accordance with the claimed method by 42,02 ± 2,63%, and in case of the active complex obtained in accordance with the closest solution - only by 27,75 ± 3,15% compared to the control level.

### References:

1. Patent MD 4296. (Ciuhrii V., "Preparation from *Tenebrio Molitor* larvae with antioxidant action, method of its obtainment and pharmaceutical preparation on its basis").
2. Patent MD 2788. (Ghicavai V., Ciuhrii M., Bacinschi N., Ciuhrii V., Chicavai V., "Entomological anti-inflammatory and antioxidant preparation").
3. Ciuhrii V., "Biotechnology of the obtainment of pharmaceutical form for the treatment of the benign prostatic hyperplasia". Dissertation for the scientific degree of the candidate of biological sciences. Chisinau, 2019, 129 pages.
4. Patent RO122332. (Ciuhrii M., "Pharmaceutical preparation in the form of rectal and vaginal suppositories for the treatment of haemorrhoids, prostate adenoma, uterine fibroids and method of its obtainment").
5. RU2117701 C1. (EKHNOLOGII VEKTOR, "Strain of Gypsy moth Lymantria Dispar L nuclear polyedrosis virus used for an insecticide preparation preparing").
6. SU577001 A1. (VNII MIKROBIOLOGICHESKIKH SRED, "Nourishing medium for cultivating caterpillars of Gypsy Moth").

## Claims

1. Method for the obtaining of an active complex from *Lymantria dispar* larvae of the 3^{rd}, 4^{th} age, that includes:
• Cultivation of *Lymantria dispar* **in** artificial conditions ;
• Freezing of larvae at -18 °C; Washing of larvae;
• Primary grinding of larvae and the obtaining of active complex;
• Lyophilisation of the active complex and the grinding of the biomass of larvae after lyophilisation, **characterized in that,** the cultivation of *Lymantria dispar* is realised on a medium with the following composition (per 1 kg of medium):
| | |
|---|---|
| Wheat flour | 80-95 g |
| Corn flour | 82-87 g |
| Dry plant material | 110-120 g |
| Skimmed milk powder | 2,5-3,5 g |
| Bee honey | 17-19 g |
| Glycerine | 10-12 g |
| Pinobanksin | 0,01-0,02 g |
| Pinocembrin | 0,01-0,02 g |
| Purified water | 696,5-663,5 ml |
and for ensures the preservation of the integral biochemical composition of the raw material and the obtaining of the product with a very high degree of homogeneity, the grinding is carried out at extremely low temperature: -55 °C, the obtained active complex being homogenized, packed and stored in conditions of refrigeration at -18 °C.

## Patentansprüche

1. Das Verfahren zur Gewinnung eines Wirkstoffkomplexes aus *Lymantria dispar*-Larven des 3. und 4. Larvenstadiums, umfassend folgende Schritte:
• Aufzucht von *Lymantria dispar* unter künstlichen Bedingungen;
• Einfrieren der Larven bei -18 °C; Waschen der Larven;
• Vorzerkleinerung der Larven und Gewinnung des Wirkstoffkomplexes;
• Einfrieren des Wirkstoffkomplexes und Zerkleinern der Larvenbiomasse nach Gefriertrocknung.
Das Verfahren **zeichnet sich dadurch aus,** dass *Lymantria dispar*-Larven auf einem Medium folgender Zusammensetzung (pro kg Medium) gezüchtet werden:
• Weizenmehl: 80-95 g
• Maismehl: 82-87 g
• Trockenes Pflanzenmaterial: 110-120 g
• Magermilchpulver: 2,5-3,5 g
• Honig: 17-19 g
• Glycerin: 10-12 g
• Pinobanksin: 0,01-0,02 g
• Pinocembrin: 0,01-0,02 g
• Gereinigtes Wasser: 696,5-663,5 ml
Um die vollständige biochemische Zusammensetzung des Rohmaterials zu erhalten und ein Produkt mit sehr hohem Homogenitätsgrad zu gewinnen, erfolgt das Mahlen bei einer extrem niedrigen Temperatur von -55 °C. Der so erhaltene Wirkstoffkomplex wird anschließend homogenisiert, verpackt und gekühlt gelagert. -18 °C.

## Revendications

1. Procédé d'obtention d'un complexe actif à partir de larves de *Lymantria dispar* des troisième et quatrième stades larvaires, comprenant les étapes consistant en
- l'élevage de l'espèce *Lymantria dispar* dans des conditions artificielle
- la congélation des larves à -18 °C ; le lavage desdites larves
- le broyage primaire des larves et l'obtention du complexe actif
- la lyophilisation du complexe actif et le broyage de la biomasse larvaire après lyophilisation, le procédé **étant caractérisé en ce que** les larves de *Lymantria dispar* sont élevées sur un milieu ayant la composition suivante (pour 1 kg de milieu) :
• Farine de blé : 80-95 g
• Farine de maïs : 82-87 g
• Matière végétale sèche : 110-120 g
• Lait écrémé en poudre : 2,5-3,5 g
• Miel d'abeilles : 17-19 g
• Glycérine : 10-12 g
• Pinobanksine : 0,01-0,02 g
• Pinocembrine : 0,01-0,02 g
• Eau purifiée : 696,5-663,5 ml
Afin d'assurer la préservation intégrale de la composition biochimique de la matière première et l'obtention d'un produit présentant un très haut degré d'homogénéité, le broyage est effectué à une température extrêmement basse, de -55 °C, le complexe actif ainsi obtenu étant ensuite homogénéisé, conditionné et stocké dans des conditions de congélation à -18 °C.
